# FASCICULE DE BREVET EUROPEEN

(11) **EP 1 890 592 B1**
(45) Date de publication et mention de la délivrance du brevet: **16.11.2011**
(21) Numéro de dépôt: 06764767.7
(22) Date de dépôt: 12.06.2006
(51) Int. Cl.: A61B 5/04

(54) **Appareil d'assistance à la cartographie per-opératoire d'aires fonctionnelles du cortex cérébral**
Gerät zum intraoperativen Mapping von Funktionsbereichen der Großhirnrinde
Assistance device for intraoperative mapping of functional areas of the cerebral cortex

(30) Priorité: 14.06.2005 FR 0505990
(43) Date de publication de la demande: 27.02.2008
(73) Titulaire: Captomed, 31670 Labege (FR)
(72) Inventeur: MONTORIOL, Pierre, F-31290 Mauremont (FR); JARRIGE, Laurent, 31410 Lavernose Lacasse (FR); BERNARDIN, Yvan, FR-31400 Toulouse (FR)
(74) Mandataire: Morelle, Guy Georges Alain
(86) Numéro de dépôt international: PCT/FR2006/001317
(87) Numéro de publication internationale: WO 2006/134254

(56) Documents cités:
- WO-A-02/45795
- WO-A-03/000128
- US-A1- 2002 022 872
- US-A1- 2003 074 032
- US-B1- 6 263 225

## Description

La présente invention appartient au domaine des instruments d'assistance opératoire dans les interventions chirurgicales, destinés en particulier à une assistance per-opératoire lors des interventions sur le cortex cérébral.

Le document US-A-2002/00522872 décrit un dispostif de stimulation du cerveau comprenant une macro-électrode de stimulation profonde de zones du cerveau et une micro-électrode rétractable de mesure du signal-réponse an niveau d'une cellule.

Depuis plusieurs années, la chirurgien du cortex cérébral a connu un essor important. Elle nécessite le recours à des techniques de cartographie permettant de distinguer les zones de lésions à éliminer des aires fonctionnelles valides à préserver. La précision du repérage est cruciale pour éviter d'endommager les zones voisines de la zone à traiter. Actuellement, la stimulation électrique directe per-opératoire du cortex cérébral s'est imposée comme la technique de cartographie la plus précise pour identifier certaines aires fonctionnelle lors de l'exérèse de lésions sous-jacentes. Pour cela, le chirurgien utilise une électrode de stimulation associée à un générateur d'impulsions simple afin de transmettre au cortex un courant électrique de faible intensité. L'excitation des neurones concernés produit un influx nerveux qui induit une activité musculaire. Cette activité, surveillée visuellement ou à l'aide d'un électromyographe, permet de délimiter les aires valides et les zones inactives.

Malgré l'intérêt évident de cette technique, celle-ci présente un inconvénient majeur qui peut être extrêmement préjudiciable à la santé de l'opéré. En effet, on a constaté que la stimulation électrique directe du cortex cérébral induit l'apparition de décharges paroxystiques hypersynchrones annonciatrices de l'arrivée très proche d'une crise épileptique. Il est alors impératif de faire cesser la crise pour pouvoir poursuivre intervention. On a pour cela généralement recours à l'irrigation du cortex à l'aide de sérum glacé, l'hypothermie diminuant le métabolisme et la diffusion électrique à l'origine de la crise. Le retour au calme n'est pas immédiat, ce qui oblige à différer la poursuite de l'intervention, du fait de l'hyperexcitabilité induite par la crise et du ralentissement du métabolisme consécutif au refroidissement.

La survenue de la crise n'est donc pas empêchée, ce qui a pour effet d'interrompre le processus de cartographie et du même coup de prolonger la durée de l'anesthésie. En outre, le sang-froid des équipes chirurgicales est mis à l'épreuve durant des interventions exigeant une haute technicité et une grande précision.

La présente invention a pour but de résoudre ce problème en proposant un appareil intégrant un dispostif de contrôle de l'activité électrique corticale. Le terme de "contrôler" sera ici employé pour désigner la double action d'appliquer une stimulation déterminée à une zone du cortex et de recueillir les signaux électriques en émanant, cette action ayant pour but de maîtriser le niveau d'excitation du cortex cérébral.

On sait que, à la suite d'une stimulation directe, l'activité électrique du cortex présente des anomalies appelées post-déchirges, apparaissant dans les secondes qui précèdent une crise épileptique. Il est possible en principe d'identifier les post-décharges signalant l'arrivée de la crise par un enregistrement de l'activité électrique du cortex. On sait également que par un phénomène de recrutement de proche en proche des neurones du cortex, le déclenchement d'une crise généralisée peut avoir pour origine une stimulation localisée. Il est donc essentiel, de détecter les signes avant-coureurs de la crise au plus près de la zone de stimulation pour espérer s'en prémunir. Il convient pour cela d'enregistrer l'activité électrique au plus près du site de stimulation et de déterminer par augmentation progressive de l'intensité de stimulation le seuil à compter duquel apparaissent les post-décharges.

C'est à partir de cette analyse qu'il a été imaginé de créer un outil permettant en un seul geste de stimuler une zone corticale et de surveiller l'activité électrique à proximité. Lorsqu'une anomalie est décelée, le chirurgien en est informé immédiatement, afin qu'il puisse intervenir sans délai de la manière là plus adaptée avant le déclenchement de la crise.

La présente invention a pour objet un appareil selon la revendication 1 intégrant un dispositif permettant de réaliser en même temps sur une même aire, une stimulation électrique et une détection de l'activité électrique corticale. Elle a également pour objet un appareil intégré permettant la surveillance automatisée de l'activité électrique corticale, grâce à des moyens d'enregistrement et de traitement des données recueillies. Elle à enfin pour objet l'application du dispositif et de l'appareil en question à la détection précoce des anomalies électriques corticales lors d'interventions chirurgicales faisant appel à la stimulation corticale directe.

La pièce à main du dispositif est définie comme la partie par laquelle on tient un instrument ou un outil, tel qu'un manche ou une poignée. Elle peut prendre toute forme et dimension la rendant compatible avec sa prise en main commode pour assurer la manipulation de l'instrument. Elle peut par exemple adopter la forme générale d'un cylindre ou d'un fuseau, ou une autre forme ergonomique. Elle est percée de part en part d'une âme permettant de laisser passer le câble conducteur reliant les électrodes aux moyens de transmission des signaux reçus et émis. L'utilisateur peut ainsi manipuler le dispositif intégré dans l'appareil selon l'invention sans risquer d'être gêné par le câble ou de l'endommager. Le câble est isolé électriquement et chacun des quatre fils est gainé séparément selon des techniques d'isolation bien connues.

Le câble conducteur est logé dans une tige rigide creuse prolongeant la pièce à main, dont l'extrémité distale supporte l'électrode de stimulation, et dont la partie médiane supporte l'électrode de recueil. Le dispositif intégré dans l'appareil selon l'invention peut ainsi être tenu et ajusté en restant à une certaine distance de la zone d'intervention, de façon à ne pas gêneur l'accès d'autres instruments ou masquer le champ utile. La tige grâce à sa rigidité; constitué un élément intermédiaire entre la pièce à main et les électrodes qui y sont associées. Elle sert en même temps au câble conducteur de conduit jusqu'aux électrodes. En effet, après avoir traversé la pièce à main et la tige creuse, deux dés fils conducteurs sont connectés à l'électrode de stimulation placée à l'extrémité distale de la tige, taudis que les deux autres fils conducteurs sont reliés à l'électrode de recueil au niveau médian de la tige.

Selon un mode de réalisation particulier, l'électrode de stimulation adopte la forme d'une fourche en matériau conducteur apte à être appliqué aux tissus corticaux. On utilise de préférence de l'acier inoxydable de qualité médicale. L'extrémité des dents de la fourche peut être une hémisphère polie.

Selon une caractéristique préférée, l'électrode de recueil est maintenue sur la tige par une liaison coulissante. Il est ainsi possible de régleur la distance séparant l'électrode de stimulation et l'électrode de recueil de façon à ce qu'elles soient toutes les deux en contact avec une aire la plus petite possible du cortex cérébral.

Selon une autre caractéristique préféré, l'électrode de recueil est maintenue sur la tige par une liaison orientable. On peut de la sorte maintenir les deux électrodes en contact avec le cortex y compris lorsque le dispositif intégré dans l'appareil selon l'invention doit être déplacé ou incliné différemment par rapport à la surface corticale.

Selon un mode de réalisation avantageux de l'invention, l'électrode de recueil est formée de deux brins en matériau conducteur apte à être mis en contact avec les tissus corticaux, lesdits brins étant maintenus sensiblement parallèles à l'axe de la tige par une barrette de liaison.

Par exemple, l'électrode de recueil peut être formée de deux brins sensiblement droits et rigides reliés chacun à la barrette de part et d'autre de la tige par une articulation pantographique stabilisée par un ressort.

Selon un autre exemple de réalisation de l'invention, l'électrode de recueil peut être formée de deux brins circulaires et souples fixés à la barrette de part et d'autre de la tige.

Dans les deux cas, les deux brins et la barrette sont avantageusement en acier inoxydable qualité médicale. Ces systèmes permettent un mouvement élastique de l'électrode par rapport à la tige (et donc aussi par rapport à l'électrode de stimulation) et par conséquent le maintien d'un parfait contact de l'électrode de recueil avec le cortex pour une inclinaison de la tige rigide d'environ 45 ± 10 degrés par rapport à la surface du cortex.

Un tel dispositif peut être utilisé pour tout type d'opération dans laquelle deux électrodes drivent être mises en contact avec un objet sur une zone de taille réduite pour stimuler et/ou recueillir un signal de manière concomitante. En particulier le dispositif intégré dans l'appareil selon l'invention trouve une application au contrôle de l'activité électrique corticale durant la cartographie per-opératoire d'aires fonctionnelles du cortex cérébral d'un patient II permet de réaliser une stimulation électrique directe du cortex cérébral tout en surveillant localement l'apparition de décharges paroxystiques hypersynchrones, annonciatrices de l'arrivée très proche d'une crise épileptique, qui sont une conséquence possible et non souhaitable de la stimulation corticale directe.

Pour une utilisation optimale du dispositif intégré dans l'appareil selon l'invention, il'est apparu indispensable de disposer d'un appareillage complet d'assistance au praticien durant les interventions chirurgicales intégrant le dispositif de contrôle de l'activité électrique corticale décrit ci-dessus et conçu pour accompagner parfaitement les avantages attendus dudit dispositif. Un des buts est notamment que l'information recueillie sur l'activité musculaire associée à une activité neuronale puisse être présentée au chirurgien en temps réel, sans distorsion, sous une forme commode telle que par exemple des signaux sonores différenciés, lui permettant d'identifier immédiatement un changement dans le niveau d'excitation de la zone de travail ou un groupe musculaire sollicité.

La stimulation électrique corticale est réalisable par exemple au moyen d'un générateur d'impulsions simples, transmises au cortex par l'électrode de stimulation associée. L'enregistrement de l'activité électrique corticale est quant à lui possible au moyen d'une électrode de type connu ou du type objet de l'invention, ou de plusieurs électrodes assemblées en réseaux, et couplées à un enregistreur d'exploration électrophysiologique.

De manière avantageuse, ledit appareil comprend d'autres accessoires périphériques ayant une fonction complémentaire du dispositif de contrôle de l'activité électrique corticale.

L'appareil selon l'invention peut comprendre des moyens pour enregistrer l'activité des muscles effecteurs associés aux aires fonctionnelles stimulées. En effet, la survenue de mouvements musculaires est une conséquence souhaitable de la stimulation électrique directe. Les capteurs susceptibles de détecter de tels mouvements sont notamment des capteurs piézo-électriques et des électrodes électromyographiques. On peut utiliser par exemple de une à huit électrodes bipolaires de recueil électromyographique. Ces électrodes sont généralement constituées de deux aiguilles subdermiques de 20 à 40 mm de longueur, séparées de 5 à 20 mm. On peut également employer de un à huit capteurs de mouvement piézo-électriques, tels que des capteurs constitués d'un disque en laiton recouvert d'un dépôt cristallin en matériau piézo-électrique dont résulte une différence de potentiel lorsque l'élément est soumis à une contrainte mécanique. Ils sont de préférence autocollants et hypo-allergéniques.

De préférence, l'appareil selon l'invention est équipé d'une électrode de référence distante, afin d'être en mesure d'éliminer le bruit de fond brouillant les signaux utiles.

Une antenne intégrée permettant la détection des hautes fréquences peut être utilement associée à l'appareil selon l'invention. En effet, les appareillages utilisés en salle d'opération comme les bistouris électriques, sont des sources importantes d'émission d'ondes perturbant le recueil des signaux utiles. En intégrant une antenne dans le système et en l'associant à des moyens de pilotage, on peut interrompre le recueil des signaux corticaux en cas de détection de hautes fréquences.

Finalement de manière préférée, l'appareil intégré selon l'invention comprend un ou plusieurs des accessoires suivants:
- au moins une électrode bipolaire de recueil électromyographique,
- au moins un capteur de mouvement piézo-électrique,
- une électrode de référence distante,
- une antenne de détection des hautes fréquences émises dans l'environnement de l'appareil.

L'unité de traitement des signaux détectés pourra traiter au choix soit uniquement les signaux issus de l'électrode de recueil de l'activité électrique corticale, soit ce qui est préférable, l'ensemble des signaux issus des électrodes corticales et des électrodes, capteurs, antennes associés à l'appareil selon l'invention.

Le générateur de signaux électriques de stimulation est de préférence un générateur de signaux rectangulaires biphasiques échantillonnés numériquement, connecté à l'électrode de stimulation. On utilise de préférence un générateur paramétrable en fréquence, intensité du courant, durée d'impulsion. Un tel générateur est de nature à garantir une parfaite adéquation temporelle du signal délivré aux paramètres programmés. Il peut être avantageusement muni par ailleurs d'un double dispositif de sécurité matériel et logiciel permettant de contrôler la valeur de consigne de l'intensité de courant, la valeur de l'intensité du courant réellement délivré, la dérive des niveaux de référence.

Selon une caractéristique importante de l'appareil selon un mode préféré de l'invention, l'unité de traitement des signaux comprend
- des moyens d'amplification différentielle des signaux issus des électrodes et des capteurs,
- des moyens de filtrage éliminant les signaux parasites,
- des moyens de numérisation des signaux filtrés.

Ces éléments sont aisément conçus par un bureau d'étude à partir d'un cahier des charges répondant aux objectifs et aux contraintes de la présente invention. Les moyens d'amplification différentielle des signaux comprennent un amplificateur différentiel multicanaux, chaque canal correspondant à une des connections issue d'une électrode ou d'un capteur.

Selon une caractéristique avantageuse, les moyens de filtrage comprennent un microcontrôleur apte à interrompre le recueil des signaux lorsque l'antenne intégrée détecte des hautes fréquences dans l'environnement de l'appareil.

Selon une autre caractéristique particulièrement intéressante de la présente invention, l'appareil d'assistance comprend des moyens de numérisation des signaux en temps réel. Il est en effet primordial que le praticien dispose des informations quant à l'état d'excitation du cortex cérébral sans délai, afin qu'il puisse prendre les mesures adéquates immédiatement et éviter le déclenchement de la crise épileptique.

De préférence, les moyens de numérisation des signaux fonctionnent selon un cadencement supérieur à 1000 Hz. Ainsi, le signal issu de chaque canal est amplifié, puis est converti en valeur numérique selon un cadencement permettant d'observer les transitoires caractéristiques des signaux surveillés, soit une fréquence d'échantillonnage supérieure à 1000 Hz. Le cadencement est supervisé en temps réel de manière à n'introduire aucune distorsion temporelle des signaux.

Selon une autre caractéristique préférée, l'appareil selon l'invention comprend des moyens de présentation en temps réel des valeurs numériques obtenues, par affichage graphique ou émission sonore. Par exemple, on peut utiliser un écran graphique en couleurs, permettant d'afficher sous forme de chronogramme déroulant, ou autre représentation graphique, les signaux observés. L'écran permet aussi d'afficher les différentes options de configuration, de réglage, d'alarmes, d'enregistrement et de sauvegarde mises à la disposition de l'utilisateur. On peut également utiliser un amplificateur audio et un haut-parleur, destinés à permettre l'écoute directe du ou des signaux recueillis. Cette écoute permet au chirurgien, dont le regard est généralement mobilisé par le microscope opératoire, d'être averti acoustiquement de la survenue de post-décharges. En effet, les signaux de post-décharge, de par leur caractère cyclique très marqué et leur intensité élevée comparativement à l'activité électrique normale du cerveau, sont aisément identifiables.

L'appareil selon l'invention peut avantageusement comprendre aussi un clavier de commandes manuelles et une unité centrale de calcul et de stockage des données nécessaires au pilotage automatique des fonctions de l'appareil et à l'enregistrement des valeurs numériques obtenues. L'unité centrale de calcul et de stockage (UCCS) est alors en charge du pilotage de l'étage de numérisation, du générateur de signaux, de l'écran. Cette unité peut être constituée d'un microprocesseur, de mémoire vive, de mémoire de masse et de différents contrôleurs de périphériques. La mémoire de masse de l'UCCS est destinée à recevoir le logiciel applicatif. Le logiciel applicatif est constitué des instructions de fonctionnement de l'UCCS.

L'appareil selon l'invention peut également comprendre en , outre des moyens d'enregistrement et de sauvegarde des valeurs numériques obtenues. Par exemple un port de communication standard permet de connecter un ordinateur ou une imprimante et ainsi de sauvegarder une séria de données enregistrée. Un logiciel applicatif installé dans l'UCCS peut permettre de réaliser l'interface homme- machine au travers de l'écran et du clavier, de commander les différentes fonctions, d'enregistrer et de stocker les données, de transmettre des données via le port de communication vers un ordinateur personnel, un réseau local ou une imprimante.

La présente invention permet de disposer d'un appareil intégré pour réaliser simultanément, sur un même site cortical et au moyen d'un seul et même équipement la stimulation électrique du cortex et l'enregistrement de l'activité électrique en découlant. On pourra également, au moyen du même appareil, enregistrer l'activité des muscles effecteurs associés aux aires fonctionnelles stimulées, par le biais de capteurs de type piézo-électrique et. électromyographique. L'information recueillie sur l'activité musculaire associée pourra être présentée au chirurgien en temps réel sous la forme de signaux sonores différenciés, lui permettant d'identifier immédiatement le groupe musculaire sollicité.

Les exemples de réalisation suivants, accompagnés de dessins, illustreront des aspects particuliers de la présente invention, sans toutefois en limiter la portée.
• La Figure 1 représente un dispositif de contrôle de l'activité électrique corticale intégré dans un appareil selon l'invention dont l'électrode de recueil est formée de deux brins sensiblement droits.
• La Figure 2 représente un dispositif de contrôle de l'activité électrique corticale intégré dans un appareil selon l'invention dont l'électrode de recueil est formée de deux brins circulaires.
• La Figure 3 est un schéma représentant un appareil d'assistance à la cartographie du cortex cérébral d'un patient

### EXEMPLE 1

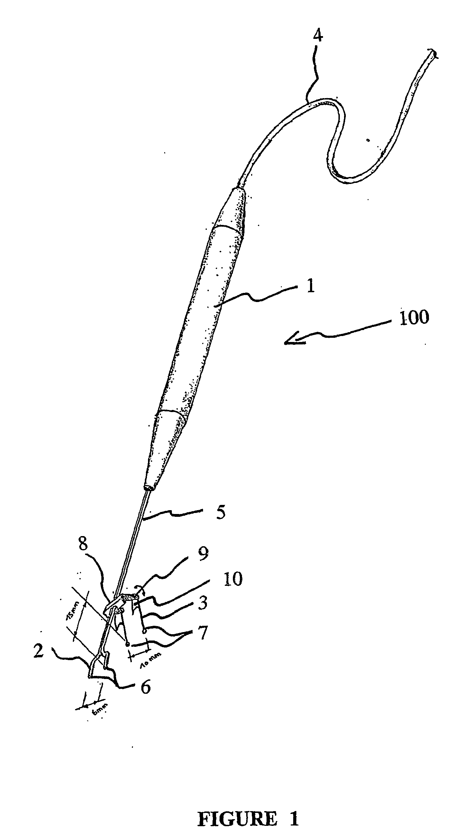

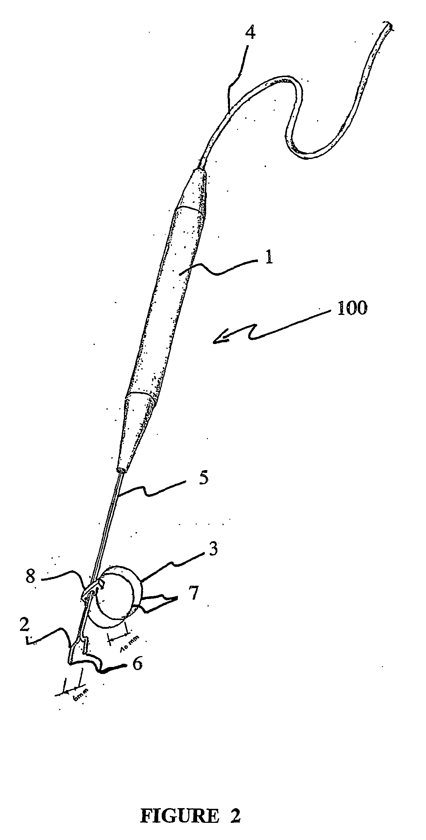

Dans un exemple illustré par les Figures 1 et 2, le dispositif 100 comprend la pièce à main 1 ergonomique en matière plastique, prolongée par la tige 5 rigide, l'électrode bipolaire de stimulation électrique 2 et l'électrode bipolaire de recueil du signal électrique 3. Les électrodes 2 et 3 sont connectées par le câble 4 traversant la pièce à main 1 à une unité chargée d'émettre et de traiter les signaux qui sera décrite plus loin. Le dispositif 100 est manipulé par le chirurgien. Ce dernier positionne la partie distale, par laquelle le courant de stimulation est délivré aux tissus, sur la zone fonctionnelle supposée.

L'électrode bipolaire de stimulation 2 est constituée de deux brins 7 en acier inoxydable de grade médical, formant une fourche. Les brins 7 ont un diamètre de 1. millimètre, et sont séparés par un entraxe de 6 millimètres. Leur extrémité est hémisphérique et polie.

L'électrode bipolaire de recueil 3 est située sur la partie médiane de la tige 5, à environ 15 mm par rapport à l'extrémité de l'électrode de stimulation 2. Sur la Figure 1, elle est maintenue sur la tige 5 par la barrette de liaison 8 coulissante et orientable. Elle est constituée de deux brins en acier inoxydable grade médical, parallèles à l'axe de la tige 5. Les brins 7 sont rigides et de forme droite, de diamètre 0,5 millimètre, séparés par un entraxe de 10. millimètres. Ils sont reliés à la tige 5 par l'articulation 9 et stabilisés par le ressort 10. Ce dispositif pantographique permet de maintenir un parfait contact de l'électrode de recueil 3 pour une inclinaison du dispositif 100 par rapport au plan d'intervention de 45 ± 10°

Sur la Figure 2, l'électrode de recueil 3 est constituée de brins 7 souples formant deux boucles. Ils sont reliés à la tige par la barrette 8 de liaison d'encastrement rigide. La garantie d'un parfait contact de l'électrode de recueil 3 pour une inclinaison du dispositif 100 par rapport au plan de travail est de 45 ± 10°, grâce à la déformation élastique réversible des boucles.

Le câble conducteur 4 est équipé d'un écran de blindage et d'un connecteur détrompé (non représentés). Il relie le dispositif 100 à l'unité d'émission et de traitement des signaux.

### EXEMPLE 2

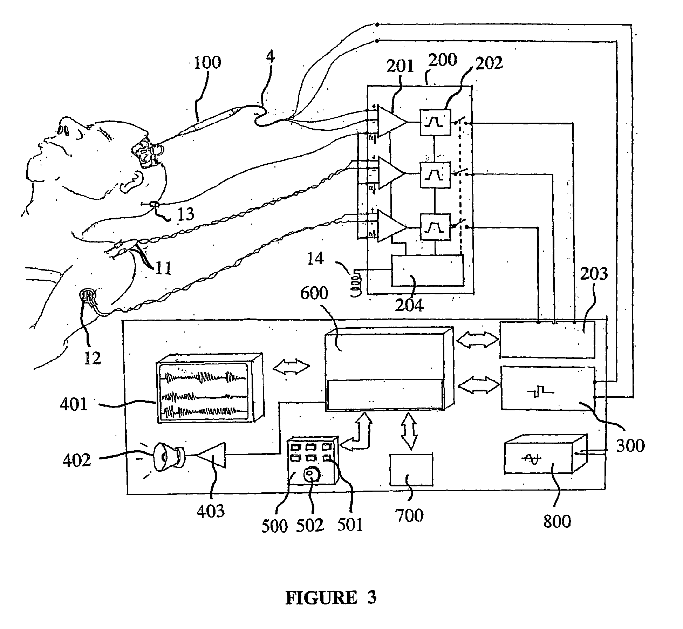

Le dispositif 100 est intégré dans un appareil d'assistance à la cartographie d'aires fonctionnelles du cortex cérébral d'un patient schématisé sur la Figure 3. Il comprend, outre le dispositif pour le contrôler de l'activité électrique corticale 100, le générateur de signaux électriques de stimulation 300 et l'unité de traitement des signaux détectés 200.

Le générateur 300 génère des signaux rectangulaires biphasiques échantillonnés numériquement. Il est paramétrable en fréquence, intensité du courant, durée d'impulsion. Les impulsions sont générées par découpage de 50 µS, garantissant une parfaite adéquation temporelle du signal délivré aux paramètres programmés. Il est muni d'un double dispositif de sécurité matériel et logiciel permettant de contrôler la valeur de consigne de l'intensité de courant, la valeur de l'intensité du courant réellement délivré, la dérive des niveaux de référence.

L'appareil représenté sur la Figure 3 comprend d'autres accessoire périphériques. Il comprend de une à huit électrodes bipolaires de recueil électromyographique 11 (une seule est ici représentée), constituées de deux aiguilles subdermiques de 20 à 40 mm de longueur, séparées de 5 à 20 mm. Leur est associée une électrode de référence distante.

Il comprend, de un à huit capteurs de mouvement piézo-électriques 12 autocollants hypo-allergéniques (un seul est ici représenté), constitués d'un disque en laiton recouvert d'un dépôt cristallin en matériau piézo-électrique dont résulte une différence de potentiel lorsque l'élément est soumis à une contrainte mécanique.

Y sont associées l'électrode de référence distante 13 et l'antenne 14 de détection des hautes fréquences émises dans l'environnement de l'appareil.

L'unité de traitement des signaux 200 est composée des amplificateurs différentiels multicanaux 201 recevant les signaux issus des électrodes et des capteurs, des moyens de filtrage 202 de signaux physiologiques, et des moyens de numérisation 203 des signaux filtrés en temps réel, selon un cadencement supérieur à 1000 Hz. Les moyens de filtrage 202 comprennent le microcontrôleur 204.

En pratique, chaque canal de l'étage d'amplification correspond soit à l'électrode de recueil 3, soit à une paire d'aiguilles de recueil électromyographique 11, soit à un capteur piézo-électrique 12. Sur chaque canal, une amplification différentielle est réalisée entre chacune des deux électrodes, puis par rapport à l'électrode de référence distante 14. Le filtrage est ensuite réalisé pour éliminer les différents signaux parasites et ne conserver que le spectre utile du signal étudié. Le microcontrôleur de supervision 204 est destiné à réaliser le contrôle de l'impédance de l'électrode de recueil 3, à détecter l'émission de hautes fréquences via l'antenne intégrée 14 et à interrompre le recueil en cas de détection de hautes fréquences.

Le signal issu de chaque canal après amplification, est transféré l'étage de numérisation 203 en temps réel des signaux, où il est converti en valeur numérique selon un cadencement supérieur à 1000 Hertz, permettant d'observer les transitoires caractéristiques des signaux surveillés. Le cadencement est supervisé en temps réel de manière à n'introduire aucune distorsion temporelle des signaux.

L'appareil selon cet exemple comprend des moyens de présentation en temps réel des valeurs numériques obtenues.

L'écran graphique 401 permet d'afficher des chronogrammes déroulants' ou autre représentation graphique les signaux observés. L'écran 401 permet aussi d'afficher les différentes options de configuration, de réglage, d'alarmes, d'enregistrement et de sauvegarde mises à la disposition de l'utilisateur.

L'amplificateur audio 403 et le haut-parleur 402 permettant l'écoute directe du ou des signaux, recueillis. Cette écoute permet au chirurgien, dont le regard est généralement mobilisé par le microscope opératoire, d'être averti acoustiquement de la survenue de post-décharges. En effet, les signaux de post-décharge, de par leur caractère cyclique très marqué et leur intensité élevée comparativement à l'activité électrique normale du cerveau, sont' aisément identifiables.

Sont également représentés le clavier de commandes 500 manuelles et l'unité centrale 600 de calcul et de stockage des données nécessaires au pilotage automatique des fonctions de l'appareil, ainsi que les moyens d'enregistrement et de sauvegarde 700 des valeurs numériques obtenues.

Le clavier 500 de commandes spécifique est constitué à la fois de touches dédiées (enclenchement de la stimulation) et de touches contextuelles 501. Le bouton rotatif numérique 502 est réservé au réglage de l'intensité de la stimulation. Le port de communication standard 800 permet de connecter l'appareil de l'invention à un ordinateur ou une imprimante et ainsi de sauvegarder une série de données enregistrée.

L'unité centrale 600 de calcul et de stockage (UCCS) est en charge du pilotage de l'étage de numérisation 203, du générateur de signaux 300, de l'écran 401. Cette unité est constituée d'un microprocesseur, de mémoire vive, de mémoire de masse et de différents contrôleurs de périphériques. La mémoire de masse de l'UCCS est destinée à recevoir le logiciel applicatif. Le logiciel applicatif est constitué des instructions de fonctionnement de l'UCCS. Il est destiné à réaliser l'interface homme-machine au travers de l'écran 401 et du clavier 500, à commander les fonctions assurées par le générateur de signaux 300, l'étage de numérisation 203, l'écran 401, le clavier 500, l'amplificateur audio 403 et le port de communication 700, à enregistrer et stocker les données, à transmettre des données via le port de communication 700 vers un ordinateur personnel, un réseau local ou une imprimante. Grâce à des messages d'avertissement, il informe l'utilisateur des éventuels risques découlant des opérations en cours. Il analyse, par des cycles permanents d'auto-vérification, le bon fonctionnement des différents organes du système. Il suspend le fonctionnement du système et en avise l'utilisateur si le dysfonctionnement d'un organe est constaté.

L'appareil est relié au réseau électrique par le bloc d'alimentation secteur 800 aux normes médicales. Il est conçu de sorte qu'un boîtier en matière plastique déporté, dénommé têtière, soit positionné au plus près du foyer chirurgical. Il intègre l'étage d'amplification différentielle et de filtrage 200 auquel seront connectées les sondes et capteurs. Il est relié au boîtier principal par un câble multiconducteur blindé. Dans le boîtier principal, en matière plastique, sont intégrées les autres fonctions.

## Revendications

1. Appareil d'assistance à la cartographie per-opératoire d'aires fonctionnelles du
cortex cérébral d'un patient comprenant un dispositif de contrôle de l'activité électrique corticale (100) comprenant une pièce à main (1) supportant :
- une électrode bipolaire de stimulation électrique (2) d'une aire du cortex,
- une électrode bipolaire de recueil du signal électrique (3) dans ladite aire,
- lesdites électrodes étant connectées chacune par une paire de fils conducteurs formant un câble (4) traversant la pièce à main (1), à un générateur de signaux électriques de stimulation (300) et à une unité de traitement des signaux détectés (200)
ledit câble conducteur (4) étant logé dans une tige (5) rigide creuse prolongeant la pièce à main (1) dont l'extrémité distale supporte l'électrode de stimulation (2), et dont la partie médiane supporte l'électrode de recueil (3).

2. Appareil selon la revendication 1, ***caractérisé en ce que*** l'électrode de stimulation (2) adopte la forme d'une fourche (6) en matériau conducteur apte à être appliqué aux tissus corticaux.

3. Appareil selon l'une des revendications 1 ou 2, ***caractérisé en ce que*** l'électrode de recueil (3) est maintenue sur la tige (5) par une liaison coulissante .

4. Appareil selon l'une des revendications 1 à 3, ***caractérisé en ce que*** l'électrode de recueil (3) est maintenue sur la tige (5) par une liaison orientable.

5. Appareil selon l'une des revendications 1 à 4, ***caractérisé en ce que*** l'électrode de recueil (3) est formée de deux brins (7) en matériau conducteur apte à être mis en contact avec les tissus corticaux, lesdits brins étant maintenus sensiblement parallèles à l'axe de la tige (5) par une barrette de liaison (8).

6. Appareil selon la revendication 5, ***caractérisé en ce que*** l'électrode de recueil (3) est formée de deux brins (7) sensiblement droits et rigides reliés chacun à la barrette (8) de part et d'autre de la tige (5) par une articulation pantographique (9) stabilisée par un ressort (10).

7. Appareil selon la revendication 5, ***caractérisé en ce que*** l'électrode de recueil (3) est formée de deux brins (7) circulaires et souples fixés à la barrette (8) de part et d'autre le la tige (5).

8. Appareil selon la revendication 1 ***caractérisé en ce qu'**il* comprend un ou plusieurs des accessoires suivants:
- au moins une électrode bipolaire de recueil électromyographique (11),
- au moins un capteur de mouvement piézo-électrique (12),
- une électrode de référence distante (13),
- une antenne (14) de détection des hautes fréquences émises dans l'environnement de l'appareil.

9. Appareil selon l'une des revendications 1 ou 8 ***caractérisé en ce que*** le générateur (300) de signaux électriques de stimulation est un générateur de signaux rectangulaires biphasiques échantillonnés numériquement, connecté à l'électrode de stimulation (2).

10. Appareil selon l'une des revendications 1, 8 ou 9 ***caractérisé en ce que*** l'unité de traitement des signaux (200) comprend :
- des moyens d'amplification différentielle (201) des signaux issus des électrodes et des capteurs,
- des moyens de filtrage (202) éliminant les signaux parasites,
- des moyens de numérisation (203) des signaux filtrés.

11. Appareil selon la revendication 10 ***caractérisé en ce que*** lesdits moyens de filtrage comprennent un microcontrôleur (204) apte à interrompre le recueil des signaux lorsque l'antenne (14) détecte des hautes fréquences dans l'environnement de l'appareil.

12. Appareil selon la revendication 10 ou 11 ***caractérisé en ce qu'**il* comprend des moyens de numérisation des signaux (203) en temps réel.

13. Appareil selon la revendication 10 à 12 ***caractérisé en ce qu'**il* comprend des moyens de numérisation des signaux (203) selon un cadencement supérieur à 1000 Hz.

14. Appareil selon l'une des revendications 8 à 13 ***caractérisé en ce qu'**il* comprend des moyens de présentation en temps réel des valeurs numériques obtenues, par affichage graphique (401) ou émission sonore (402).

15. Appareil selon l'une quelconque des revendications 8 à 14 ***caractérisé en ce qu'***il comprend un clavier de commandes (500) manuelles et une unité centrale (600) de calcul et de stockage des données nécessaires au pilotage automatique des fonctions de l'appareil.

16. Appareil selon l'une quelconque des revendications 8 à 15 ***caractérisé en ce qu'***il comprend en outre des moyens d'enregistrement et de sauvegarde (700) des valeurs numériques obtenues.

## Claims

1. Appliance for assisting in the intraoperative mapping of functional areas of the cerebral cortex of a patient comprising a device for monitoring the cortical electrical activity (100) comprising a handheld piece (1) supporting:
- a bipolar electrode (2) for electrically stimulating an area of the cortex,
- a bipolar electrode (3) for collecting the electrical signal in said area,
- said electrodes each being connected by a pair of conductive wires forming a cable (4) passing through the handheld piece (1), with an electrical stimulation signal generator (300) and with a unit (200) for processing the detected signals,
said conductive cable (4) being housed in a hollow rigid rod (5) in extension of the handheld piece (1) whose distal end supports the stimulation electrode (2), and whose medium portion supports the collection electrode (3).

2. Appliance according to Claim 1, ***characterized in that*** the stimulation electrode (2) adopts the shape of a fork (6) made of conductive material that can be applied to the cortical tissues.

3. Appliance according to one of Claims 1 or 2, ***characterized in that*** the collection electrode (3) is held on the rod (5) by a sliding link.

4. Appliance according to one of Claims 1 to 3, ***characterized in that*** the collection electrode (3) is held on the rod (5) by an orientable link.

5. Appliance according to one of Claims 1 to 4, ***characterized in that*** the collection electrode (3) is formed by two strands (7) made of conductive material that can be placed in contact with the cortical tissues, said strands being held substantially parallel to the axis of the rod (5) by a link bar (8).

6. Appliance according to Claim 5, ***characterized in that*** the collection electrode (3) is formed by two substantially straight and rigid strands (7) each linked to the bar (8) on either side of the rod (5) by a pantographic articulation (9) stabilized by a spring (10).

7. Appliance according to Claim 5, ***characterized in that*** the collection electrode (3) is formed by two circular and flexible strands (7) fixed to the bar (8) on either side of the rod (5).

8. Appliance according to Claim 1, ***characterized in that*** it comprises one or more of the following accessories:
- at least one bipolar electromyographic collection electrode (11),
- at least one piezoelectric motion sensor (12),
- a remote reference electrode (13),
- an antenna (14) for detecting high frequencies emitted in the environment of the appliance.

9. Appliance according to one of Claims 1 or 8, ***characterized in that*** the electrical stimulation signal generator (300) is a generator of digitally sampled two-phase rectangular signals, connected to the stimulation electrode (2).

10. Appliance according to one of Claims 1, 8 or 9, ***characterized in that*** the signal processing unit (200) comprises:
- means (201) for differentially amplifying the signals from the electrodes and the sensors,
- filtering means (202) eliminating the spurious signals,
- means (203) for digitizing the filtered signals.

11. Appliance according to Claim 10, ***characterized in that*** said filtering means comprise a microcontroller (204) that can interrupt the collection of the signals when the antenna (14) detects high frequencies in the environment of the appliance.

12. Appliance according to Claim 10 or 11, ***characterized in that*** it comprises means (203) for digitizing the signals in real time.

13. Appliance according to Claims 10 to 12, ***characterized in that*** it comprises means (203) for digitizing the signals at a rate higher than 1000 Hz.

14. Appliance according to one of Claims 8 to 13, ***characterized in that*** it comprises means for presenting in real time the digital values obtained, by graphical display (401) or sound emission (402).

15. Appliance according to any one of Claims 8 to 14, ***characterized in that*** it comprises a manual control keypad (500) and a central unit (600) for computing and storing the data needed to automatically drive the functions of the appliance.

16. Appliance according to any one of Claims 8 to 15, ***characterized in that*** it also comprises means (700) for recording and backing up the digital values obtained.

## Patentansprüche

1. Vorrichtung zur Unterstützung des intraoperativen Mapping von Funktionsbereichen der Großhirnrinde eines Patienten mit einer Vorrichtung zur Steuerung der elektrischen kortikalen Aktivität (100), die ein Handstück (1) aufweist, das
- eine bipolare Elektrode zur elektrischen Stimulierung (2) eines Bereichs der Hirnrinde und
- eine bipolare Elektrode zur Aufnahme des elektrischen Signals (3) in dem Bereich trägt,
- wobei die Elektroden jeweils durch ein Paar leitender Drähte, die ein das Handstück (1) durchquerendes Kabel (4) bilden, mit einem Generator von elektrischen Stimulationssignalen (300) und einer Einheit zur Verarbeitung der erfassten Signale (200) verbunden sind,
wobei das Leiterkabel (4) in einem starren, hohlen Stift (5) aufgenommen ist, der das Handstück (1) verlängert, dessen distales Ende die Stimulationselektrode (2) trägt und dessen mittlerer Teil die Aufnahmeelektrode (3) trägt.

2. Vorrichtung nach Anspruch 1, ***dadurch gekennzeichnet, dass*** die Stimulationselektrode (2) die Form einer Gabel (6) aus einem leitenden Material annimmt, die an kortikale Gewebe anlegbar ist.

3. Vorrichtung nach einem der Ansprüche 1 oder 2, ***dadurch gekennzeichnet, dass*** die Aufnahmeelektrode (3) durch eine verschiebbare Verbindung auf dem Stift (5) gehalten ist.

4. Vorrichtung nach einem der Ansprüche 1 bis 3, ***dadurch gekennzeichnet, dass*** die Aufnahmeelektrode (3) durch eine schwenkbare Verbindung auf dem Stift (5) gehalten ist.

5. Vorrichtung nach einem der Ansprüche 1 bis 4, ***dadurch gekennzeichnet, dass*** die Aufnahmeelektrode (3) von zwei Einzeldrähten (7) aus leitendem Material gebildet wird, die mit den kortikalen Geweben in Kontakt bringbar sind, wobei die Einzeldrähte durch einen Verbindungssteg (8) im Wesentlichen parallel zur Achse des Stiftes (5) gehalten werden.

6. Vorrichtung nach Anspruch 5, ***dadurch gekennzeichnet, dass*** die Aufnahmeelektrode (3) durch zwei im Wesentlichen gerade und starre Einzeldrähte (7) gebildet ist, die jeweils auf beiden Seiten des Stiftes (5) durch ein mittels einer Feder (10) stabilisiertes pantographisches Gelenk (9) mit dem Steg (8) verbunden sind.

7. Vorrichtung nach Anspruch 5, ***dadurch gekennzeichnet, dass*** die Aufnahmeelektrode (3) von zwei kreisförmigen und nachgiebigen Einzeldrähten (7) gebildet wird, die auf beiden Seiten des Stifts (5) an dem Steg (8) befestigt sind.

8. Vorrichtung nach Anspruch 1, ***dadurch gekennzeichnet, dass*** sie ein oder mehrere der folgenden Zubehörteile aufweist:
- wenigstens eine elektromyografische bipolare Aufnahmeelektrode (11),
- wenigstens einen piezoelektrischen Bewegungssensor (12),
- eine entfernte Referenzelektrode (13),
- eine Antenne (14) zur Erfassung von in die Umgebung der Vorrichtung abgegebenen Hochfrequenzen.

9. Vorrichtung nach einem der Ansprüche 1 oder 8, ***dadurch gekennzeichnet, dass*** der Generator (300) von elektrischen Stimulationssignalen ein Generator von rechteckigen, biphasischen, digitalisierten Signalen ist, der mit der Stimulationselektrode (2) verbunden ist.

10. Vorrichtung nach einem der Ansprüche 1, 8 oder 9, ***dadurch gekennzeichnet, dass*** die Signalverarbeitungseinheit (200)
- Einrichtungen zur Differentialverstärkung (201) der von den Elektroden und den Sensoren abgegebenen Signale,
- Filterungseinrichtungen (202) zur Eliminierung von parasitären Signalen,
- Einrichtungen (203) zur Digitalisierung der gefilterten Signale umfasst.

11. Vorrichtung nach Anspruch 10, ***dadurch gekennzeichnet, dass*** die Filterungseinrichtungen einen Mikrocontroller (204) aufweisen, der die Aufnahme der Signale unterbrechen kann, wenn die Antenne (14) Hochfrequenzen in der Umgebung der Vorrichtung erfasst.

12. Vorrichtung nach Anspruch 10 oder 11, ***dadurch gekennzeichnet, dass*** sie Einrichtungen zur Digitalisierung der Signale (203) in Realzeit aufweist.

13. Vorrichtung nach Anspruch 10 bis 12, ***dadurch gekennzeichnet, dass*** sie Einrichtungen zur Digitalisierung der Signale (203) in einer Frequenz von mehr als 1000 Hz aufweist.

14. Vorrichtung nach einem der Ansprüche 8 bis 13, ***dadurch gekennzeichnet, dass*** sie Einrichtungen zur Präsentation der erhaltenen digitalen Werte in Realzeit durch grafische Anzeige (401) oder Tonabgabe (402) aufweist.

15. Vorrichtung nach einem der Ansprüche 8 bis 14, ***dadurch gekennzeichnet, dass*** sie eine Tastatur für manuelle Steuerungen (500) und eine Zentraleinheit (600) zum Berechnen und Speichern der für die automatische Steuerung der Funktionen der Vorrichtung erforderlichen Daten aufweist.

16. Vorrichtung nach einem der Ansprüche 8 bis 15, ***dadurch gekennzeichnet, dass*** sie außerdem Einrichtungen zum Aufzeichnen und Speichern (700) der erhaltenen digitalen Werte aufweist.
